Europäisches Patentamt

European Patent Office

Office européen des brevets

(11) Publication number: 0 349 955
A1

## EUROPEAN PATENT APPLICATION

(21) Application number: 89112121.2

(51) Int. Cl.⁴: A61B 17/34

(22) Date of filing: 03.07.89

(30) Priority: 05.07.88 IT 2123188

(43) Date of publication of application:
10.01.90 Bulletin 90/02

(84) Designated Contracting States:
DE ES FR GB

(71) Applicant: Pianetti, Francesco
Via Turati, 23
I-20013 Magenta (Milano)(IT)

(72) Inventor: Pianetti, Francesco
Via Turati, 23
I-20013 Magenta (Milano)(IT)

(74) Representative: Gervasi, Gemma et al
NOTARBARTOLO & GERVASI Srl Viale
Bianca Maria 33
I-20122 Milan(IT)

(54) Laparoscopy trocar.

(57) Laparoscopy trocar having a conical screw shaped point (3) suitable for insertion by screwing down. The use of such trocar permits to avoid lesions of endoabdominal organs and vessels, which occurs when using needles known in the art.

fig 1

EP 0 349 955 A1

# LAPAROSCOPY TROCAR

## Field of the invention

The present invention pertains to a laparoscopy trocar suitable for insertion by screwing down.

## Prior technique

In the laparoscopy technique cylindrical needles are normally used having a diameter of 9.5 mm and a conical or pyramidal point. When using such needles it is necessary to adopt particular precautions so as to avoid dangerous accidents, such as lesions of endoabdominal organs and vessels.

Therefore, when carrying out a laparoscopy, a pneumoperitoneum is first effected, by introducing into the peritoneal cavity 3 to 4 l $CO_2$.

The pneumoperitoneum brings about a lifting of the abdominal anterior wall moving away the viscera from it; in particular, in case the patient is placed in a slight Trendelenburg's position, the viscera will tend to move to the upper abdominal region.

It is necessary, however, to accurately control the endoabdominal pressure due to the pneumoperitoneum. The pressure must, first of all, be kept below 20-30 mm of mercury, to avoid compression of the vena cava and of the diaphragm, as in the first case the venous drainage would be impeded, and in the second case a vasovagal reflex could be expected, accompanied by Arhythmia, bradycardia, hypertension and albeit exceptionally, cardiac arrest.

On the other end, the endoabdominal pressure should not be too low because when the trocar is inserted, the abdominal wall should remain lifted in order to minimize the risk of causing lesions to the endoabdominal organs and vessels.

The trocar should be inserted at an angle of 45° in periumbilical lower site, after having sectioned the skin in a semicircle around the lower border of the umbilicum and the subcutaneous tissue, to reach the aponeurosis. The trocar should be pushed with caution, avoiding an abrupt and deep penetration which could cause lesions of the viscera and large vessels.

Even if all these precautions are used, however, it isn't always possible to avoid traumatic events in the cited anatomical structures and complications connected with the pneumoperitoneum as such.

At present, mortal events occur at the rate of one death on 10,000 to 100,000 operations, depending on the statistical sources.

Among the non-lethal events we may mention:

1) Omentum hemorrhages with necessity of surgical hemostasia: 5%;

2) Hemorrhages of umbilical vessels: 1%;

3) Intestine, stomach or bladder lesions: 0,1%;

4) Heart or circulation alterations: 1%;

5) Lesions to large vessels: 3 to 9 on 10,000.

Informations on such events and their determination are supplied, for instance, by Hoffer, F.D. and Pent, D.Statistics in Phillips, J.M., ed: Laparoscopy William & Wilkins, Baltimore, 1977.

## Summary of the invention

The inconveniences in the laparoscopy performed according to the known technique are overcome through the use of the trocar according to the present invention, which is characterized by having a conical screw shaped point which allows the insertion by screwing down.

## Detailed description of the invention

The characteristics and the advantages of the laparoscopy trocar according to the invention will be further illustrated in the following detailed description.

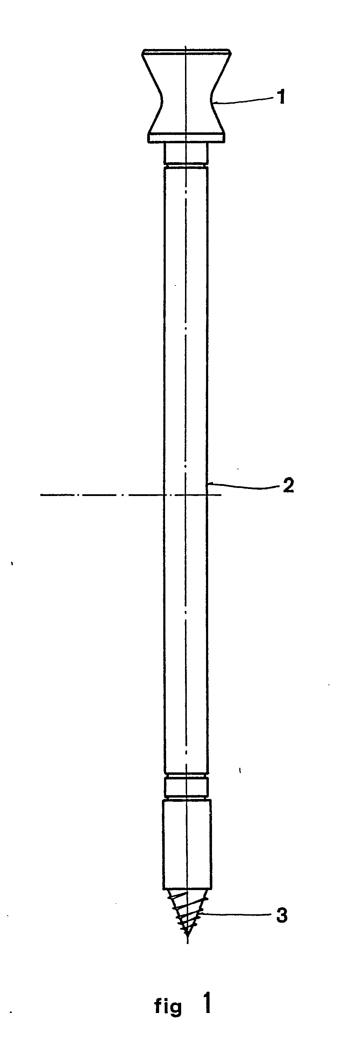

The needle according to the invention is represented in figure 1. It comprises a head (1), a body (2) and a point (3). The head and the body have the same conformation as in the known technique, and in particular the body (2) has a cylindrical shape with a diameter of 9,5 mm.

The point has the characteristic shape of a conical screw which can be obtained by threading with a constant pitch of 2 mm or by a spiroid increasing progressively toward the base of the cone.

The taper of the point (3) is the same as in the known technique.

The use of the trocar according to the invention in the praxis of laparoscopy allows to operate without exerting any compression on the aponeurotic fascia, because the insertion of the needle takes place easily by screwing down. In this way the needle penetrates without depressing the abdominal wall, avoiding the risk of causing lesions to the organs mentioned.

It is furthermore possible to noticeably reduce the pressure of the pneumoperitoneum in view of the fact that the trocar, entering as a screw, does not exert any pression and does not require any resistance on the part of the wall.

In this way, all the complications caused by the endoabdominal pressure of the pneumoperitoneum are avoided. As a consequence, through the use of the trocar according to the invention, the following basic advantages are obtained:

1) As the trocar enters the abdominal cavity as a screw and without any pressure whatever, the traumatic lesions to the viscera and large vessels are avoided.

In fact, it is not necessary to push the needle against the abdominal wall, which, if depressed, would come dangerously near to the underlaying vascular structure (cava and aorta).

2) The complications caused by endoabdominal pressure in the pneuimoperitoneum are avoided; the pneumoperitoneum can be kept at a very low pressure and not as a complement to the tension of the abdominal wall due to the penetration of the point of the needle.

## Claims

1. A laparoscopy trocar characterized in that its point is in the shape of a conical screw apt to be inserted by screwing down.

2. A trocar according to claim 1, characterized in that said conical screw is obtained by threading with a constant pitch of 2 mm.

3. A trocar according to claim 1, characterized in that said screw is obtained through a spiroid increasing progressively toward the base of the cone.

fig 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | US-A-4 191 191 (AUBURN) <br> * Column 2, lines 41-47; figures 2,3 * <br> --- | 1-3 | A 61 B 17/34 |
| A | FR-A-2 350 821 (BAYLIS) <br> * Page 4, lines 18-21; figure 9 * <br> ----- | 1 | |
| | | | **TECHNICAL FIELDS SEARCHED (Int. Cl.5)** |
| | | | A 61 B |

The present search report has been drawn up for all claims

| Place of search | Date of completion of the search | Examiner |
|---|---|---|
| THE HAGUE | 25-09-1989 | MOERS R.J. |

EPO FORM 1503 03.82 (P0401)